# EUROPEAN PATENT SPECIFICATION

(11) **EP 1 251 882 B1**
(45) Date of publication and mention of the grant of the patent: **23.08.2006**
(21) Application number: 00914517.8
(22) Date of filing: 04.02.2000
(51) Int. Cl.: A61L 9/014, A61L 9/12

(54) **AIR DEODORIZATION DEVICE WITH A DETACHABLE CARTRIDGE**
VORRICHTUNG MIT EINER LÖSBAREN PATRONE ZUM DESODORIEREN VON LUFT
DISPOSITIF DESODORISANT D'AIR A CARTOUCHE AMOVIBLE

(43) Date of publication of application: 30.10.2002
(73) Proprietor: THE PROCTER & GAMBLE COMPANY, Cincinnati, Ohio 45202 (US)
(72) Inventor: STIROS, Paul, Cincinnati, OH 45243 (US); KVIETOK, Frank, Andrej, Cincinnati, OH 45208 (US)
(74) Representative: Peet, Jillian Wendy
(86) International application number: PCT/US2000/002907
(87) International publication number: WO 2001/056621

(56) References cited:
- FR-A- 2 189 076
- US-A- 5 772 738
- DATABASE WPI Section Ch, Week 199524 Derwent Publications Ltd., London, GB; Class D14, AN 1995-181605 XP002148973 & JP 06 288672 A (GOLDSTAR CO LTD), 18 October 1994 (1994-10-18) -& JP 06 288672 A (GOLDSTAR CO LTD) 18 October 1994 (1994-10-18)
- DATABASE WPI Section Ch, Week 199151 Derwent Publications Ltd., London, GB; Class A88, AN 1991-373442 XP002148972 & JP 03 251253 A (DAISO CO LTD), 8 November 1991 (1991-11-08)

## Description

### Field of the invention

The present invention relates to air deodorizing devices for removing malodor from the air. Such devices are useful for example for storing and preserving food in closed compartments such as refrigerators.

### Background

Nowadays, refrigerators have become a common appliance in virtually every household and typically are used for storage and preservation of food, in particular of fresh food such as fruits, vegetables, dairy products, and the like. It is desirable to keep the food items fresh as long as possible in the refrigerator.

It is a well known problem that many food items tend to release malodors into the air which are then captured in the limited air space in a refrigerator. Not only are these malodors unpleasant and offensive to the user of the refrigerator, they can also have a negative impact on the quality of other foods in the refrigerator. For example, it is known that some foods emit strong odors (e.g. fish, boiled eggs, onions, etc.) and that these odors can transfer to other nearby foods and hurt the taste and freshness of those foods. A common example is transfer of odors into an open container of orange juice or of milk resulting in a noticeable degradation in their taste. It is also well known that malodors from some vegetables (onions, garlic) can transfer to other foods stored within a vegetable drawer. This problem is aggravated when the vegetable drawer is sealed such that there is very little air exchange with the larger compartment of the refrigerator (herein referred to as the "fresh food compartment") and when vegetables have been cut or are stored without any outer wrapping. This problem of odor transfer is particularly acute in the case of ice cubes where odors from the fresh food compartment of the refrigerator can be transferred to the ice in the freezer compartment of the refrigerator. This is especially true in the case of refrigerators in which there is air exchange between the fresh food and freezer compartments, and especially in the case of refrigerators with built-in ice-makers.

U.S. patent No. 5,403,548 discloses an activated carbon absorbent to be used for example in refrigerators, shoe boxes, closets, toilets, cars, cupboard, or the like. The activated carbon absorbent is applied in a gas treating apparatus comprising an air inlet, an air outlet, a cylinder housing the activated carbon honeycomb, and a fan aspiring malodor through the air inlet. Change of battery and withdrawal of the cylinder housing the activated carbon are achieved by dismounting the cover of the apparatus. The gas treating apparatus may further comprise an action member for alerting the user to the event that the useful life of the activated carbon adsorbent has run out. This gas treating apparatus has the disadvantages that exchange of batteries and withdrawal of the activated carbon require a multitude of steps including dismounting of the cover and that exchange of battery and activated carbon are carried out separate from each other.

It is therefore an object of the present invention to provide an air deodorizing device which overcomes the disadvantages of the prior art devices.

It is an object of the present invention to provide an air deodorizing device comprising a replaceable cartridge member housing a filter member whereby the cartridge member is directly accessible from the outside.

It is a further object of the present invention to provide an air deodorizing device comprising a cartridge member housing a replaceable filter member whereby the cartridge member is directly accessible from the outside.

It is a further object of the present invention to provide an air deodorizing device comprising a replacement cartridge member housing a power supply and a filter member.

It is a further object of the present invention to provide an air deodorizing device having a replaceable power supply and a replaceable filter member, the lifetimes of the power supply and the filter member being of the same magnitude.

### Summary of the invention

The present invention provides an air deodorizing device comprising a cartridge member, the air deodorizing device having an air flow path from an air inlet to an air outlet. The cartridge member comprises a filter member and is arranged with the filter element in interaction with the air flowing along the air flow path. The air deodorizing device further comprises an air moving member for moving air along the air flow path, and is characterized in that the cartridge member is detachable from the air deodorizing device and directly accessible from the outside.

### Detailed description of the invention

The device of the present invention is intended to deodorize air, in particular air in confined compartments such as refrigerators, trash bins, cars, closets, and the like.

The cartridge member of the present invention housing the filter member is detachable from the device. The term "detachable" as used herein refers to members which can be easily removed, in particular where no tools such as screw drivers are needed. Preferably, no excessive forces are need for detaching the cartridge means, the cartridge means is directly accessible from the outside, and the cartridge member can be removed by holding the device of the present invention in one hand and by removing the cartridge member with the other hand.

Deodorization of the air in the device of the present invention is achieved by adsorbing the molecules constituting a malodor onto a surface of a filter member. The term "adsorption" is well defined in the art and refers to the adherence of molecules to surfaces which effectively reduces the mobility of these molecules to the two dimensions of the surface. Those molecules remaining in the air will then diffuse so that further molecules come into contact with the surface and subsequently will be adsorbed. Consequently, most of the malodor molecules will travel into the proximity of one of the surfaces at some point in time so that finally most of the malodor will be removed from the air.

A suitable filter member comprises activated carbon for the adsorption. Activated carbon is known to be a very effective filter medium due to its high specific surface area. Whilst activated carbon is very effective as such, the filter member of the present invention may further comprise agents supported on the activated carbon to specifically attack certain malodors such as those comprising S atoms or N atoms. A wide variety of activated carbon based filter media is known in the art. Preferably, the filter members of the present invention comprise at least 2 grams, more preferably at least 5 grams, and most preferably at least 10 grams of activated carbon. Preferably, the filter members of the present invention comprise less than 100 grams, more preferably less than 50 grams, yet more preferably less than 40 grams, and most preferably less than 30 grams of activated carbon.

The filter member of the present invention comprises an air inlet, an air outlet, and an air flow path through the filter member from the air inlet to the air outlet. The filter medium is disposed in the filter member of the present invention such that it comes into contact with the air flowing along the air flow path. The filter medium may be arranged as a flow by filter or as a flow through filter. The filter member of the present invention may comprise a support for the filter medium for example in the form of a foam, a nonwoven material or a woven material. Preferably, the activated carbon is supported on a polyurethane foam having an activated carbon density of at least 0.01 grams per cm³, more preferably of at least 0.05 grams per cm³, and most preferably of at least 0.1 grams per cm³ and having an activated carbon density of less than 0.3 grams per cm³.

The deodorization of the air in the device of the present invention is enhanced by increasing the air flow through the filter member by means of an air moving member. Preferably, the air moving means moves at least 100 ml of air per second through the air inlet into the device, more preferably at least 200 ml/s, most preferably at least 300ml/s. There are known in the art a wide variety of suitable air moving members such as for example fans and blowers. A particularly suitable fan is a centrifugal fan. A suitable member for driving the fan is a small motor, for example a DC motor available from MABUCHI MOTOR CO.,LTD., Japan, under the designation of RF-330TK. The air moving members of the present invention may be powered electrically. Many electrical power sources could be imagined including domestic AC electrical power or power from a static power supply. Alternatively and preferably electrical power may be supplied by means of a battery, preferably a dry alkaline cell battery, or a rechargeable battery. The electrical power may also be received from a solar cell. Any replaceable power supply preferably is designed to last at least one month, more preferably at least two months, yet more preferably at least three months, most preferably at least four months.

To improve the malodor removal performance and to simplify the mechanical construction of the air deodorizing device of the present invention, the filter member and the air moving member are preferably arranged such that substantially all air aspired by the air moving member is forced to flow through the filter member before it penetrates the air inlet of the air moving member. In other words, the air inlet of the cartridge member is unitary with the air inlet of the air deodorizing device. In this setup, only one air path connection is needed between the fitter member and the air member and hence complexity is decreased. Furthermore, withdrawal of the cartridge member is greatly simplified if only one connection has to be disengaged. Any disengageable air flow connection may of course comprise sealing members to improve air flow performance. Any such connection may further comprise a mechanical engaging members to stabilize the connection.

The filter member of the present invention may be replaced by detaching the cartridge member from the device of the present invention and inserting in a new one. It is to be understood in this context that the present invention includes embodiments in which the cartridge member as a whole comprising a filter member is replaced and further includes embodiments in which the cartridge is reused and contains a replaceable filter element.

The cartridge member of the present invention is directly accessible from the outside of the device without the need to unmount any covers or the like. More preferably, the cartridge member can simply be lifted off from the device. For example, the cartridge member may simply be sitting on top of the air moving member only held in place by gravitational forces whereby the surface topology of the interfacing parts of the cartridge member and the air moving member match each other such as in a hemispherical design.

Optionally, the cartridge member of the present invention may further comprise the power supply for the air moving member. In this case, the interface between the cartridge member and the air moving member needs to comprise electrical contacts connecting the power supply with the drive member of the air moving member. In this context, it is to be understood, that the

Preferably, the lifetime of the filter member and the lifetime of the power supply are substantially equal so that both members may be replaced at the same time intervals. In this case, an empty power supply would also signal that the filter element has to be replaced.

The air deodorizing device of the present invention may comprise a signal member to indicate if the life span of the filter member has run out. The signal member may be slaved to the time the filter member has been exposed to air or may be slaved to the overall runtime of the air moving member. In case the power supply for the air moving member is included in the filter member and accordingly is replaced with the filter member, the signal member may be slaved to the remaining charge of the power supply thus indicating that filter member and power supply needs to be replaced.

## Claims

1. An air deodorizing device comprising a cartridge member, said air deodorizing device having an air flow path from an air inlet to an air outlet, said cartridge member comprising a filter member and being arranged with
said filter element in interaction with the air flowing along said air flow path,
said air deodorizing device further comprising an air moving member for
moving air along said air flow path,
**characterized in that**
said cartridge member is detachable from said air deodorizing device, and wherein said cartridge member is directly accessible from the outside of said device.

2. An air deodorizing device according to claim 1
wherein
said air deodorizing device is intended to be used for refrigerators.

3. An air deodorizing device according to claim 2
wherein said cartridge member sits on top of the air moving member, preferably stabilized through a matching of shaped surfaces.

4. An air deodorizing device according to claim 1,
said air moving member being powered by a replaceable power supply
wherein
said cartridge member comprises said replaceable power supply, said replaceable power supply preferably being designed to last at least 1 month,
the lifetime of said power supply member and the lifetime of said filter member preferably being substantially equal.

5. An air deodorizing device according to claim 1
wherein
said air moving member for moving air along the air flow path is a fan.

6. An air deodorizing device according to claim 1
wherein
said air moving member is electrically driven and is powered by a battery.

7. An air deodorizing device according to claim 1
wherein
said filter member comprises activated carbon.

8. An air deodorizing device according to claim 7
wherein
said activated carbon is supported on a support member, said support member comprising a foam, a woven, or a nonwoven web material.

9. An air deodorizing device according to claim 8
wherein activated carbon is supported on a polyurethane foam having a carbon density of between 0.01 and 0.3 grams per cm³.

10. An air deodorizing device according to claim 7
wherein
said filter member comprises between 5 and 30 grams of activated carbon.

11. An air deodorizing device according to claim 1,
said cartridge member having an air flow path, said air moving member having an air inlet and an air outlet
wherein
set filter member and said air moving member are arranged such that substantially all air aspired into said air moving member is forced to flow through said cartridge member along said air flow path before said air penetrates through said air inlet into said air moving member.

## Patentansprüche

1. Lufterfrischungsgerät, das ein Patronenelement umfasst, wobei das Lufterfrischungsgerät einen Luftstromweg von einem Lufteinlass zu einem Luftauslass aufweist, wobei das Patronenelement ein Filterelement umfasst und mit dem Filterelement in Wechselwirkung angeordnet ist, wenn die Luft den Luftstromweg entlang strömt, wobei das Lufterfrischungsgerät ferner ein Luftbewegungselement umfasst, um Luft den Luftstromweg entlang zu bewegen, **dadurch gekennzeichnet, dass** das Patronenelement von dem Lufterfrischungsgerät abgenommen werden kann, und wobei das Patronenelement direkt von außerhalb des Geräts zugänglich ist.

2. Lufterfrischungsgerät nach Anspruch 1, wobei das Lufterfrischungsgerät zur Verwendung für Kühlschränke vorgesehen ist.

3. Lufterfrischungsgerät nach Anspruch 2, wobei das Patronenelement über dem Luftbewegungselement sitzt und vorzugsweise durch eine Übereinstimmung geformter Oberflächen stabilisiert wird.

4. Lufterfrischungsgerät nach Anspruch 1, wobei das Luftbewegungselement mit einer austauschbaren Stromquelle betrieben wird, wobei das Patronenelement die austauschbare Stromquelle umfasst, wobei die austauschbare Stromquelle vorzugsweise so ausgelegt ist, dass sie mindestens 1 Monat hält, wobei die Lebensdauer des Stromquellenelements und die Lebensdauer des Filterelements vorzugsweise im Wesentlichen gleich sind.

5. Lufterfrischungsgerät nach Anspruch 1, wobei das Luftbewegungselement zum Bewegen von Luft den Luftstromweg entlang ein Ventilator bzw. Gebläse ist.

6. Lufterfrischungsgerät nach Anspruch 1, wobei das Luftbewegungselement elektrisch angetrieben und durch eine Batterie betrieben wird.

7. Lufterfrischungsgerät nach Anspruch 1, wobei das Filterelement Aktivkohle umfasst.

8. Lufterfrischungsgerät nach Anspruch 7, wobei die Aktivkohle auf einem Trägerelement aufgebracht ist, wobei das Trägerelement ein Bahnenmaterial aus Schaumstoff, Gewebe oder Vliesstoff umfasst.

9. Lufterfrischungsgerät nach Anspruch 8, wobei Aktivkohle auf einem Polyurethanschaum aufgetragen ist und eine Kohlenstoffdichte zwischen 0,01 und 0,3 Gramm pro cm³ aufweist.

10. Lufterfrischungsgerät nach Anspruch 7, wobei das Filterelement zwischen 5 und 30 Gramm Aktivkohle umfasst.

11. Lufterfrischungsgerät nach Anspruch 1, wobei das Patronenelement einen Luftstromweg aufweist, wobei das Luftbewegungselement einen Lufteinlass und einen Luftauslass aufweist, wobei das Filterelement und das Luftbewegungselement so angeordnet sind, dass im Wesentlichen die gesamte in das Luftbewegungselement eingesogene Luft gezwungen wird, den Luftstromweg entlang durch das Patronenelement zu strömen, bevor die Luft durch den Lufteinlass in das Luftbewegungselement dringt.

## Revendications

1. Dispositif désodorisant d'air comprenant un élément à cartouche, ledit dispositif désodorisant d'air ayant une voie de circulation d'air allant d'une entrée d'air à une sortie d'air, ledit élément à cartouche comprenant un élément filtrant et étant disposé de telle sorte que l'élément filtrant soit en interaction avec l'air circulant le long de ladite voie de circulation d'air, ledit dispositif désodorisant d'air comprenant en outre un élément de déplacement d'air destiné à déplacer l'air le long de ladite voie de circulation d'air, **caractérisé en ce que** ledit élément à cartouche est amovible dudit dispositif désodorisant d'air, et où ledit élément à cartouche est directement accessible à partir de l'extérieur dudit dispositif.

2. Dispositif désodorisant d'air selon la revendication 1, où ledit dispositif désodorisant d'air est prévu pour être utilisé dans des réfrigérateurs.

3. Dispositif désodorisant d'air selon la revendication 2, dans lequel ledit élément à cartouche est situé au-dessus de l'élément de déplacement d'air, de préférence stabilisé à l'aide de surfaces de formes correspondantes.

4. Dispositif désodorisant d'air selon la revendication 1, ledit élément de déplacement d'air étant alimenté par une alimentation électrique remplaçable où ledit élément à cartouche comprend ladite alimentation électrique remplaçable, ladite alimentation électrique remplaçable étant de préférence conçue pour durer au moins 1 mois, la durée de vie dudit élément d'alimentation électrique et la durée de vie dudit élément filtrant étant de préférence sensiblement égales.

5. Dispositif désodorisant d'air selon la revendication 1, dans lequel l'élément de déplacement d'air destiné à déplacer l'air le long de la voie de circulation d'air est un ventilateur.

6. Dispositif désodorisant d'air selon la revendication 1, dans lequel ledit élément de déplacement d'air est entraîné électriquement et est alimenté par une batterie.

7. Dispositif désodorisant d'air selon la revendication 1, dans lequel ledit élément filtrant comprend du charbon actif.

8. Dispositif désodorisant d'air selon la revendication 7, dans lequel ledit charbon actif est supporté sur un élément de support, ledit élément de support comprenant un matériau de nappe en mousse, tissé, ou non tissé.

9. Dispositif désodorisant d'air selon la revendication 8, dans lequel le charbon actif est supporté sur une mousse polyuréthane ayant une masse volumique de carbone allant de 0,01 à 0,3 grammes par cm³.

10. Dispositif désodorisant d'air selon la revendication 7, dans lequel ledit élément filtrant comprend entre 5 et 30 grammes de charbon actif.

11. Dispositif désodorisant d'air selon la revendication 1, ledit élément à cartouche ayant une voie de circulation d'air, ledit élément de déplacement d'air ayant une entrée d'air et une sortie d'air où ledit élément filtrant et ledit élément de déplacement d'air sont disposés de sorte que sensiblement tout l'air aspiré dans ledit élément de déplacement d'air soit forcé à traverser ledit élément à cartouche le long de ladite voie de circulation d'air avant que ledit air ne pénètre par ladite entrée d'air dans ledit élément de déplacement d'air.
